# EUROPEAN PATENT SPECIFICATION

(11) **EP 2 983 656 B1**
(45) Date of publication and mention of the grant of the patent: **27.11.2019**
(21) Application number: 14730995.9
(22) Date of filing: 09.04.2014
(51) Int. Cl.: A61K 31/132, A61P 17/00, A61P 29/00, A61K 8/41, A61Q 19/08

(54) **COMPOSITION FOR RESISTING SKIN AGEING THROUGH AN ANTI-INFLAMMATORY ACTION**
ZUSAMMENSETZUNG ZUR BEKÄMPFUNG VON HAUTALTERUNG DURCH EINE ENTZÜNDUNGSHEMMENDE WIRKUNG
COMPOSITION DESTINÉE À EMPÊCHER LE VIEILLISSEMENT DE LA PEAU PAR UNE ACTION ANTI-INFLAMMATOIRE

(30) Priority: 09.04.2013 IT MI20130555
(43) Date of publication of application: 17.02.2016
(73) Proprietor: Giuliani S.p.A., 20129 Milano (IT)
(72) Inventor: GIULIANI, Giammaria, 20123 Milano (IT); BENEDUSI, Anna, 20124 Milano (IT); MARZANI, Barbara, 27020 Carbonara Al Ticino (IT); BARONI, Sergio, 24030 Villa D'Adda (IT); PAUS, Ralf, 22339 Hamburg (DE)
(74) Representative: Coppo, Alessandro
(86) International application number: PCT/IB2014/060555
(87) International publication number: WO 2014/167508

(56) References cited:
- WO-A1-99/37277
- WO-A1-2005/013932
- ROBERT A. CASERO ET AL: "Treatment with ?-difluoromethylornithine plus a spermidine analog leads to spermine depletion and growth inhibition in cultured L1210 leukemia cells", JOURNAL OF CELLULAR PHYSIOLOGY, vol. 121, no. 3, 1 December 1984 (1984-12-01), pages 476-482, XP055090095, ISSN: 0021-9541, DOI: 10.1002/jcp.1041210305
- DATABASE CA [Online] CHEMICAL ABSTRACTS SERVICE, COLUMBUS, OHIO, US; 23 August 1986 (1986-08-23), KITAURA, YOSHIHIKO ET AL: "Spermidine derivatives", XP002728881, retrieved from STN Database accession no. 1986:460484 & JP 61 053250 A (FUJISAWA PHARMACEUTICAL CO., LTD., JAPAN) 17 March 1986 (1986-03-17)
- OYANAGUI, YOSHIHIKO ET AL: "Steroid-like anti- inflammatory effects of arcaine and spermidine analogs", POLYAMINES: BASIC CLIN. ASPECTS, PROC. SATELL. SYMP. ( 1985 ), MEETING DATE 1984, 311-18. EDITOR(S): IMAHORI, KAZUTOMO. PUBLISHER: VNU SCI. PRESS, UTRECHT, NETH. CODEN: 54HBAT, 1985, pages 311-318, XP008171512,

## Description

### FIELD OF THE INVENTION

The present invention relates to a cosmetic or pharmaceutical composition for resisting the effects of skin ageing.

### BACKGROUND OF THE INVENTION

The human skin is constantly exposed to air, to solar radiation, to environmental pollutants, physical and chemical agents that are able to induce the production of free radicals in the body.

Oxidative damage made by free radicals is a main cause of physical ageing in general, and of the skin in particular. The ageing process consists in a deterioration of metabolic balance, or homeostasis, accompanied by an alteration of the physiological systems, including the immune system.

One of the targets of oxidative damage within the cell is mitochondrion, which generates most of the energy for cellular requirements and which consumes more than 90% of the oxygen. The process of mitochondrial oxidation is therefore directly associated with ageing.

The changes in the immune function related to advancing age originate from oxidative and inflammatory stress. Numerous studies show that chronic inflammation is the primary responsible for skin ageing.

The two major pro-inflammatory stimuli are the alteration or destruction of the skin barrier and ultraviolet radiation. In general, the environmental insults inducers of free radicals lead to a destruction of the skin barrier that activates the release of TNF-alpha and interleukins IL-1 and IL-8, as well as other pro-inflammatory molecules that induce an acute inflammatory reaction. Acute inflammation does not seem to be an inducer of direct damage to the skin, but it aggravates skin ageing in progress.

On the other hand, the repeated alteration of the skin barrier, even if slight, activates chronic inflammation.

Considering this evidence, it seemed possible to slow or delay the skin ageing process by acting on chronic inflammation with treatments based on antioxidants and anti-inflammatory products.

Although at the skin level there are endogenous systems of protection from free radicals, such as melanin and some enzyme and non antioxidant systems, it must be considered that skin antioxidants defense mechanisms during the ageing process decreases physiologically.

This physiological decline adds up to a further important depletion of the pool of endogenous antioxidants due to UV exposure. Faced with this reduction, many studies point out that topical treatment with antioxidants is useful in restoring the defenses against free radicals, in some cases even reversing the ageing process.

WO2005013932 by the same Applicant describes a composition for pharmaceutical, dieting or cosmetic use for human use adapted to slow down the ageing of the skin and skin annexes, including spermine, spermidine or salts thereof as active principle. In particular, this document describes experimental studies related to the effects of such polyamines on the elasticity, hydration and the cell renewal action of the skin. However, no effects or anti-inflammatory or antioxidants are described or suggested therein by the active compounds spermine, spermidine or salts thereof.

It should also be noted that in the case of topical formulations, spermidine, as well as other polyamines, is subject to oxidation because when applied topically on the skin, before being absorbed by the skin to perform its action, it remains for some time in extended contact with air. A possible oxidation of spermidine during that time before the absorption would lead to oxidation products that are no longer active.

JP 61 053250 A (Fujisawa Pharmaceutical Co. Ltd; Japan) discloses a N¹-phenylspermidine and other spermidine derivatives as inflammation inhibitors. This document does not disclose an anti-inflammatory effect on the skin.

According to the present invention, it has now been surprisingly found that the effects of skin ageing may be resisted through an anti-inflammatory action using a compound of general formula (I): R-N¹-spermidine as defined below, as such or in the form of a pharmaceutically acceptable derivative such as a salt.

### DESCRIPTION OF THE INVENTION

The object of the invention are compounds of formula (I) R-N¹-spermidine, i.e. 1.4-butanediamine, N-(3-aminopropyl)-N¹-R,

(I) H₂N-(CH₂)₃-N¹(R)-(CH₂)₄-NH₂

wherein R is a substituent bound to the secondary amine function of spermidine, selected from:
methyl, ethyl, propyl, isobutyl,
or a corresponding pharmaceutically acceptable salt,
for use in opposing the effects of skin ageing through an anti-inflammatory action.

As shown in the experimental part below in the present description, such anti-inflammatory activity is also accompanied by an antioxidant activity against oxygen free radicals (ROS), comparable with that of α-tocopherol.

Moreover, the compounds of general formula (I) are both active, and stable to air so as to allow an efficient application for topical use on the skin without being transformed into a different, not active substance due to the effect of oxidation.

Such compounds of general formula (I), being therefore provided with an anti-inflammatory activity and slowing the skin ageing processes, can be effectively used for topical application on the skin in the form of suitable compositions for cosmetic or pharmaceutical use for the achievement of such effects.

Therefore, the invention also provides a composition for use according to claim 9 and compounds of formula (I) for use as defined in claim 17. The composition herein disclosed is adapted to carry out an anti-inflammatory effect and slowing the skin ageing processes and containing at least one compound of formula (I) as active principle as such or in the form of a pharmaceutically acceptable derivative such as a salt, for topical administration on the skin.

### DETAILED DESCRIPTION OF THE INVENTION

Suitable forms for topical use are, for example, a cream, a serum, a balm, a mask, a gel for the face or the body.

If said compound of formula (I) is in the form of a pharmaceutically acceptable derivative such as a salt, it is preferably a maleic acid salt, such as trimaleate, or a hydrochloric acid salt, such as trichydrochloride.

Any other organic or inorganic acid salt pharmaceutically acceptable for a formulation for topical application is suitable.

A preferred compound of formula (I) for use according to claim 4 is N¹-methylspermidine, or N-(3-aminopropyl)-N¹-methyl-1,4-butanediamine (CAS Registry Number 51460-23-2), of formula:

(II) H₂N - (CH₂)₃-N¹(CH₃)-(CH₂)₄-NH₂

and for use in a composition according to claim 9 or 10 as such or as a pharmaceutically acceptable salt, such as trimaleate (3C₄H₄O₄) or trihydrochloride (3HCl).

Further preferred compounds of formula (I) for use according to claim 5, 6 or 7, respectively, are:
N¹-ethylspermidine, of formula

   (IV) H₂N-(CH₂)₃-N¹(C₂H₅)-(CH₂)₄-NH₂
N¹-propylspermidine, of formula

   (V) H₂N-(CH₂)₃-N¹(C₃H₇)-(CH₂)₄-NH₂
N¹-isobutylspermidine, of formula

   (VI) H₂N-(CH₂)₃-N¹(C₄H₉)-(CH₂)₄-NH₂

A compound of formula (I) for use according to claim 1, as such or in the form of pharmaceutically acceptable derivative such as a salt, is contained in a composition in an amount preferably within the following ranges in percentage by weight, w/w (%): 0.0001 to 0.30; preferably from 0.010 to 0.30 and from 0.0001 to 0.15.

Compositions for use according to the invention formulated for topical use on the skin of the body or face are described hereinafter, by way of examples.

The amounts of the components, indicated by the INCI nomenclature, are expressed as a percentage by weight variable within the ranges indicated therein.

### Example 1

### CONCENTRATED FACIAL SERUM

**Component (INCI name) Amount w/w (%)**

| | |
|---|---|
| Citric acid | 0.001-0.30 |
| Sodium hydroxide | 0.001-0.30 |
| Gellan gum | 0.10-0.80 |
| Xanthan gum | 0.01-0.80 |
| Sorbic acid | 0.01-0.20 |
| Phenoxyethanol | 0.10-0.99 |
| Sorbitol | 0.10-1.00 |
| N¹-Methyl-spermidine | 0.010-0.30 |
| Disodium EDTA | 0.025-0.10 |
| Caprylyl glycol | 0.01-0.30 |
| Limnanthes alba seed oil | 0.20-5.00 |
| Paraffinum liquidum | 0.05-0.90 |
| C12-20 Alkyl glucoside | 0.10-0.99 |
| C14-22 Alcohols | 0.10-0.99 |
| Glycerin | 0.50-8.00 |
| Propanediol | 0.50-8.00 |
| Hydroxylethyl acrylate/Sodium acryloyldimethyl taurate copolymer | 0.05-1.00 |
| Polyisobutene | 0.01-0.90 |
| PEG-7 Trimethylolpropane Coconut Ether | 0.01-0.90 |
| Parfum | 0.10-0.70 |
| Aqua | as needed 100 g |

### Example 2

### LOTION FOR SKIN AND SCALP

| | |
|---|---|
| Alcohol | 15-20 |
| Calcium pantothenate | 1-2 |
| PEG-40 Hydrogenated castor oil | 0.75-1.5 |
| Lactic acid | 0.1-0.5 |
| Parfum | 0.1-0.2 |
| Hydroxypropyltrimonium hyaluronate | 0.02-0.08 |
| N¹-Ethylspermidine | 0.02-0.08 |
| Octadecyl di-t-Butyl-4-hydroxyhydrocinnamate | 0.02-0.08 |
| Lecithin | 0.02-0.08 |
| Rutin | 0.001-0.003 |
| Biotin | 0.001-0.003 |
| Vitis vinifera seed extract | 0.005-0.01 |
| Polyurethane-26 | 0.002-0.008 |
| Helianthus annuus seed oil | 0.002-0.004 |
| Olea europaea leaf extract | 0.0005-0.001 |
| Zeaxanthin | 0.0005-0.001 |
| Aqua | as needed to 100 |

### Example 3

### HYDRATING SERUM

**Component (INCI name) Amount w/w (%)**

| | |
|---|---|
| N¹-Methyl-spermidine | 0.0001-0.15 |
| Xanthan gum | 0.02-0.50 |
| Sodium alginate | 0.01-0.50 |
| Gellan gum | 0.10-0.80 |
| Citric acid | 0.001-0.30 |
| Sodium hydroxide | 0.001-0.30 |
| Glycerin | 0.50-5.00 |
| Panthenol | 0.01-0.20 |
| Sorbitol | 0.50-5.00 |
| Propanediol | 0.50-5.00 |
| Penthylene glycol | 0.50-4.00 |
| Potassium sorbate | 0.05-0.20 |
| Sodium benzoate | 0.05-0.20 |
| Parfum | 0.050-0.50 |
| Peg 40-hydrogenated castor oil | 0.10-0.80 |
| PEG-6-Caprylic/Capric Glycerides | 0.10-0.80 |
| Aqua | as needed 100 g |

### Example 4

### MICELLAR WATER

**Component (INCI name) Amount w/w (%)**

| | |
|---|---|
| Glycerin | 1-10 |
| Cocoyl proline | 1-2 |
| Coco-glucoside | 0.5-1.5 |
| Gluconolactone | 0.05-0.15 |
| N¹-Propylspermidine | 0.1 |
| Sodium lauroyl sarcosinate | 0.1-1 |
| Phenoxyethanol | 0.1-1 |
| Sodium benzoate | 0.1-0.5 |
| Sodium hydroxide | 0.1-0.2 |
| Citrus Aurantium Amara flower water | 0.05-0.15 |
| Disodium EDTA | 0.05-0.15 |
| Sorbityl furfural | 0.05-0.2 |
| Calcium gluconate | 0.05-0.1 |
| Sodium chloride | 0.01-0.05 |
| Parfum | 0.01-0.02 |
| Sodium chloride | 0,005-0.02 |
| Tocopheryl acetate | 0.005-0.015 |
| Sodium benzoate | 0,001-0,005 |
| Potassium sorbate | 0,001-0,005 |
| Benzyl alcohol | 0.001-0.002 |
| Aqua | as needed to 100 |

### Example 5

### NOURISHING MOISTURIZING FACIAL CREAM

**Component (INCI name) Amount w/w (%)**

| | |
|---|---|
| Xanthan gum | 0.05-0.20 |
| Disodium EDTA | 0,025-0.05 |
| Xilitol | 0.50-1.50 |
| Glycerin | 0.50-3.00 |
| Sorbitol | 0.50-3.00 |
| Trehalose | 0.50-3.00 |
| Cyclopentasiloxane | 0.10-3.00 |
| Sodium hydroxymethylglycinate | 0.05-0.30 |
| Limnanthes alba seed oil | 0.20-5.00 |
| Paraffinum liquidum | 0.50-5.00 |
| Caprylic/capric triglyceride | 0.50-5.00 |
| Butyrospermum parkii butter | 0.10-0.50 |
| Isostearyl isostearate | 0.50-5.00 |
| Glyecryl stearate | 0.20-3.00 |
| Sucrose cocoate | 0.20-3.00 |
| Polyglyceryl-3 Rice Branate | 0.50-4.00 |
| Dimethicone | 0.20-6.00 |
| Cetearyl alcool | 0.20-5.00 |
| Phenoxyethanol | 0.30-0.90 |
| N¹-Methyl-spermidine | 0.0001-0.15 |
| Parfum | 0.10-0.30 |
| Lactic acid | 0.001-0.30 |
| Aqua | as needed 100 g |

### Example 6

### SKIN CLEANSING SOLUTION

**Component (INCI name) Amount w/w (%)**

| | |
|---|---|
| Monoethanolamine laurysulphate | |
| Ricinoleth-40 | 5-15 |
| DEA derivatives of phosphatides from soya lecithin | 1.5-7.5 |
| Phenoxyethanol | 0.2-0.5 |
| N¹-Isobutylspermidine | 0.01-0.3 |
| Lactic acid sol. 80 | 0.02-0.08 |
| Perfume | 0.01-0.05 |
| Aqua | as needed to 100 |

### Example 7

### AFTER SUN BODY MILK

**Component (INCI name) Amount w/w (%)**

| | |
|---|---|
| Glycerin | 1.00-6.00 |
| Methylpropanediol | 1.00-6.00 |
| Cetyl hydroxyethylcellulose | 0.10-0.40 |
| Xanthan gum | 0.10-0.40 |
| Tapioca starch | 1.00-2.00 |
| Disodium EDT A | 0.025-0.20 |
| Sorbitan stearate | 2.00-5.00 |
| Sucrose cocoate | 0.10-1.00 |
| Ethylexyl palmitate | 1.00-5.00 |
| Hydrogenated polydecene | 1.00-5.00 |
| Caprilic/capric triglycerides | 1.00-5.00 |
| Butyrospermum parkii | 1.00-5.00 |
| Limnanthes alba seed oil | 1.00-3.00 |
| N¹-Methyl-spermidine | 0.0001-0.15 |
| Dimethicone | 1.00-3.00 |
| Sodium hydroximethylglycinate | 0.10-0.20 |
| Phenoxyethanol | 0.70-0.90 |
| Lactic acid | as needed |
| Parfum | 0.30 |
| Delta tocopherol | 0.02-0.25 |
| Sorbityl furfural | 0.10-0.90 |
| Aqua | as needed 100.00 |

### Example 8

### FACIAL TONER

**Component (INCI name) Amount w/w (%)**

| | |
|---|---|
| Glycerin | 0.50-6.00 |
| Butylene glycol | 0.50-8.00 |
| Methylpropanediol | 0.50-6.00 |
| N¹-Methyl-spermidine | 0.0001-0.15 |
| Hydroxyethylcellulose | 0.02-0.80 |
| Polysorbate 20 | 0.50-10.00 |
| PEG 60-almond glycerides | 0.10-1.00 |
| PEG-8 | 0.10-4.00 |
| Butylene glycol | 0.50-8.00 |
| Phenoxyethanol | 0.10-0.90 |
| Citric acid | 0.001-0.30 |
| Sodium hydroxide | 0.001-0.30 |
| Disodium EDTA | 0.05-0.10 |
| Parfum | 0.05-0.40 |
| Benzoic acid | 0.01-0.20 |
| Aqua | as needed 100 g |

### Example 9

### MAKEUP REMOVER

**Component (INCl name Amount w/w %**

| | |
|---|---|
| C12-15 Alkyl benzoate | 5.00-30.00 |
| Hydrogenated polydecene | 5.00-30.00 |
| Limnanthes alba seed oil | 0.50-10.00 |
| N¹-Methyl-spermidine | 0.0001-0.15 |
| Sodium chloride | 0.01-0.40 |
| Disodium EDTA | 0.001-0.09 |
| Magnesium sulfate | 0.01-0.40 |
| Citric acid | 0.01-0.40 |
| Sodium hydroxide | 0.01-0.40 |
| Glycerin | 0.50-6.00 |
| Sodium methylparaben | 0.020-0.20 |
| Sodium ethylparaben | 0.020-0.20 |
| Aqua | as needed 100 g |

### Reference Example 10

### SOOTHING SKIN LOTION

**Component (INCI name) Amount w/w (%)**

| | |
|---|---|
| Glyceryl stearate palmitate | 5-15 |
| Glyceryl stearate palmitate | 2-8 |
| Coconut oil | 1-5 |
| Cetostearyl alcohol | 1-5 |
| Emulsifying wax | 1-5 |
| Benzyl alcohol | 0.5-1.5 |
| N¹-Cyclohexylspermidine | 0.2 |
| Cetyl alcohol | 0.02-0.06 |
| Aqua | as needed to 100 |

### EXPERIMENTAL TESTS

The following tests were carried out in order to study the activity of the compounds of formula (I) for the use according to the present invention.

### DESCRIPTION OF THE DRAWINGS

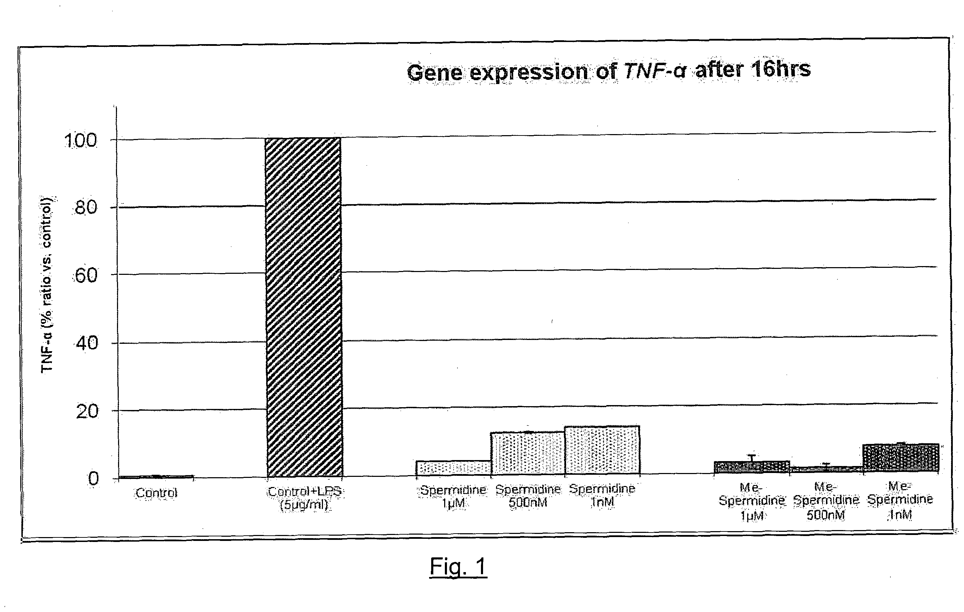

The results of these tests are shown in the graphs in Figures 1 to 5 of the accompanying drawings, as described below.

### ANTI-INFLAMMATORY TEST

With reference to Figures 1, 2 and 3, samples of a compound of the invention, N¹ - methylspermidine, i.e. N-(3-aminopropyl)-N¹-methyl-1,4-butanediamine of formula (II) as defined above, were subjected to the following anti-inflammatory test, using samples of spermidine as a reference comparison.

### Culture and propagation of the cell line

An immortalized line of human keratinocytes NCTC2544 is used (Perry, V.P., Sanford, K.K., Evans, V.J. , Hyatt, G.W., Earle, W.R., 1957: Establishment of clones of epithelial cells from human skin. J Natl Cancer Inst. 18 (5): 709-717) cultured in sterile flasks (25 cm³), incubated at 37 °C in a humid atmosphere at 5% CO₂ in MEM (Minimum Essential Medium) added with bovine fetal serum (FBS), 2 mm L-glutamine, 1% non-essential amino acids, in the presence of 1% penicillin and streptomycin.

A 1: 3 split is done every 2 days upon achieving the monolayer by washing with 1 x PBS (phosphate buffer without Ca²⁺ and Mg²⁺) and detachment of cells with a trypsin-EDTA solution at 37 °C for 2 minutes.

### Control

Negative control: human keratinocytes NCTC2544 cultured in EMEM (EBSS) at 2.5% FBS, supplemented with 1% L-glutamine 2mM, 1% solution of amino acids and 1% mixture of penicillin (10,000 U/ml)/streptomycin (10,000 Ug/ml) at 37 °C, 5% CO₂.

Positive control: human keratinocytes NCTC2544 treated with lipopolysaccharide (LPS, 5µg/ml) in EMEM (EBSS) at 2.5% FBS, supplemented with 1% L-glutamine 2mM, 1% solution of amino acids and 1% mixture of penicillin (10,000 U/ml)/streptomycin (10,000 Ug/ml) at 37 °C, 5% CO₂.

### Methods

The gene expression of TNF-α on NCTC2544 cells treated with the samples was evaluated by qRT-PCR.

The analysis of gene expression involves four steps:
1. Treatment of cells with the samples for 16 and 24 hours;
2. RNA extraction;
3. Retrotranscription in cDNA;
4. Quantitative RT-PCR.

### Treatment of NCTC2544 cells

In the experimental conditions, in relation with the results obtained in a previous MTT test, samples of spermidine and N¹-methyl-spermidine were tested at the following concentrations: 1 µM, 500 nM and 1 mM (final concentration in the medium).

Said positive and negative controls were also tested.

### Anti-inflammatory test on NCTC2544

### Day 1: cell seeding

When the human keratinocyte cells NCTC 2544 have reached about 80% confluence, they are detached with trypsin/EDTA and seeded at a density of 1×10⁶cells/ml in 12-well plates and incubated at 37 °C, 5% CO₂ (24h).

### Day 2: exposure to the samples for 16 and 24 h

The above samples of spermidine and N¹-methyl-spermidine were dissolved in EMEM, supplemented with 2.5% FCS, 1% 2 mM L-glutamine, 1% NEAA solution and 1% penicillin (10,000 U/ml)/streptomycin (10,000 pg/ml).

The controls, containing only culture medium (negative control) and culture medium plus LPS (5µg/ml) (positive control), were included in each plate.

The cells were exposed to the action of the samples of spermidine and N¹-methyl-spermidine at the above concentrations of 1 µM, 500 nM and 1 mM: a total of six samples.

LPS at a concentration of 5 µg/ml was added to each well (except in the negative control). Each sample was tested in replicate.

### Results

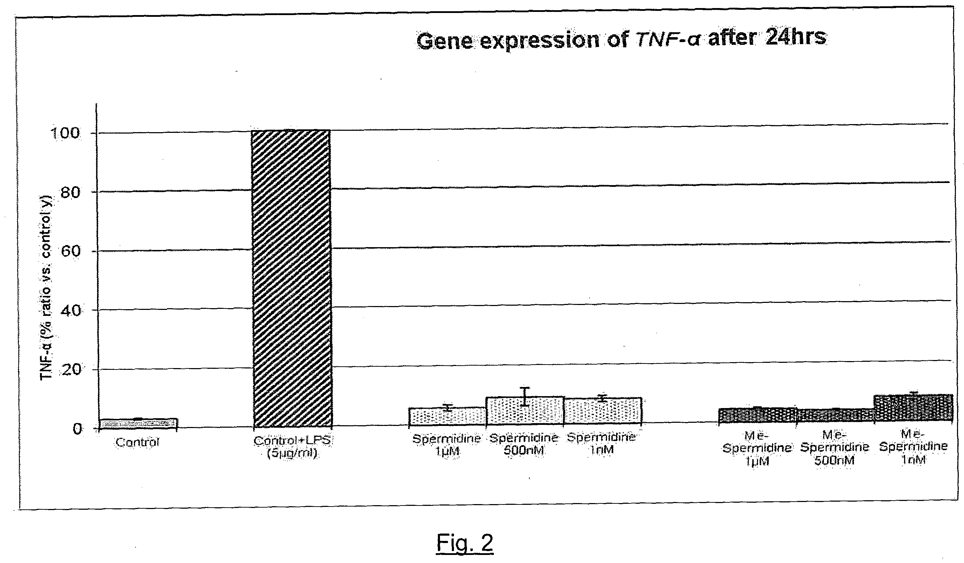

The results are shown in the graphs of Figures 1 and 2 of the accompanying drawings, wherein N¹-methylspermidine is indicated with the abbreviation 'Me-spermidine' compared with 'spermidine' taken as a reference.
Fig. 1 shows the gene expression of TNF-α on NCTC2544 cells treated with the six samples in question and the two controls as defined above, after 16 hours of treatment.
Fig. 2 shows a similar diagram after 24 hours of treatment.

In general, the results obtained show an anti-inflammatory effect of N¹-methyl-spemidine at all concentrations examined.

In Fig. 1, after 16 hours of treatment, at the concentration of 1 µM the anti-inflammatory effect of spermidine and N¹-methyl-spermidine, although being better for the compound of the invention with respect to the comparison, can be said to be comparable (4:29% and 3:25%, respectively); however, at lower concentrations of 500 nM and 1 nM there was a significantly greater anti-inflammatory effect for N¹-methyl-spermidine than for spermidine, with a TNF-α expression equal to 1.52% at 500nm and 7.94% at 1 nM in the case of N¹-methylspermidine, against 12:48% at 500 nm, and 13.99% at 1 nM in the case of spermidine.

In particular, the improved anti-inflammatory effect is remarkable at the concentration of 500 nM.

In Fig. 2, after 24 hours the anti-inflammatory effects of N¹-methyl-spermidine remain almost unchanged, even if compared to Fig. 1 they are decreased at the concentration of 500 nM, for which, however, there is still a significantly higher anti-inflammatory effect of N¹-methyl-spermidine than spermidine.

### ROS ANTIOXIDANT TEST

Samples of a compound of the invention, N¹-methylspermidine trihydrochloride salt, i.e. N-(3-aminopropyl)-N¹-methyl-1,4-butanediamine 3HCl, were subjected to the following antioxidant test to reactive oxygen species (ROS).

The production of ROS was monitored spectrofluorometrically using 2',7'-dichlorofluorescein diacetate (DCFH-DA), as described by Tobi et al. (Tobi SE, Paul N, T McMillan J. J Photochem Photobiol, B 2000: 57: 102-112).

The NCTC 2544 cells (about 80% confluence) were detached with trypsin/EDTA and seeded at a density of 5x10⁴ cells per well in 96-well plates. Subsequently, the cells were treated with samples of N¹-methylspermidine, at the following concentrations: 500 nM, 1 µm and 500 µm, 1 mM (final concentration in the culture medium).

α-tocopherol (250 µg/ml, 580 µm) was used as a comparison of the antioxidant activity. The plates were incubated at 37 °C in 5% CO₂ for 1.5 hours. Cells cultured on basal medium with 2.5% FBS were used as a control.

### Results

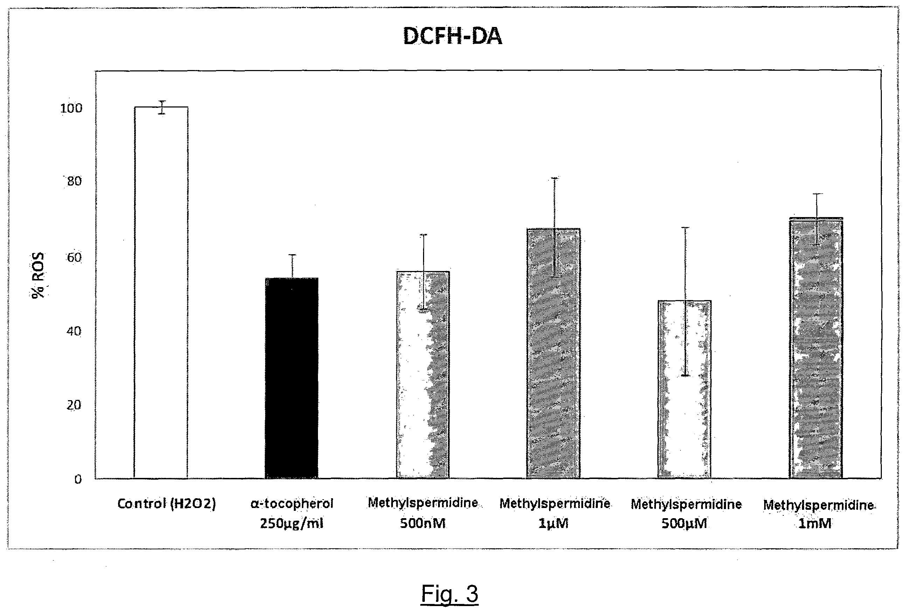

The results are shown in the graph of Fig. 3, wherein N¹-methylspermidine trichydrochloride salt is for brevity indicated as 'methylspermidine'. The ordinate shows the decrease in the production of ROS (ROS%) compared to the control (H₂O₂, ROS = 100%) for N¹-methylspermidine at the various concentrations indicated therein and for α-tocopherol.

Compared with the control cells treated with H₂O₂ both cells treated with α-tocopherol and those treated with the compound of the invention at the various concentrations indicated therein showed a decrease substantially comparable to the intracellular concentration of ROS produced. In particular, it may be seen that at the concentration of 500 µm, N¹-methylspermidine trichydrochloride salt a shows a higher antioxidant activity than that of α-tocopherol (580 µM).

From the complex of the experimental evidence summarized above, it is noted that the compounds of formula (I) are suitable for a pharmaceutical or cosmetic use aimed at resisting the effects of skin ageing mainly through an anti-inflammatory action.

Such anti-inflammatory activity is also accompanied by an antioxidant activity against oxygen free radicals (ROS) comparable with that of α-tocopherol.

Moreover, the compounds of general formula (I) are both active according to the purposes of the present invention, and stable to air so as to allow an efficient application for topical use on the skin without being transformed into a different, not active substance due to the effect of oxidation.

### ANTI-INFLAMMATORY TEST

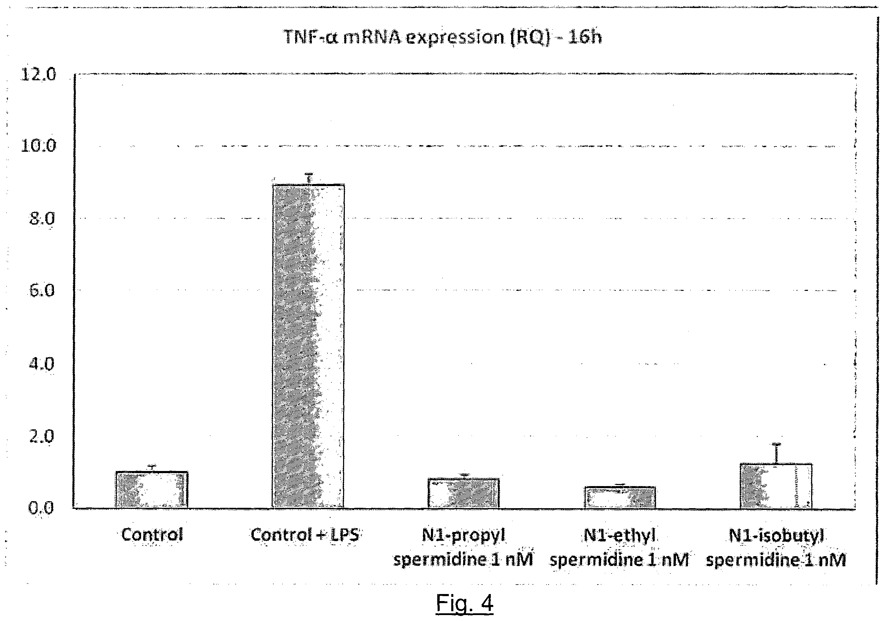

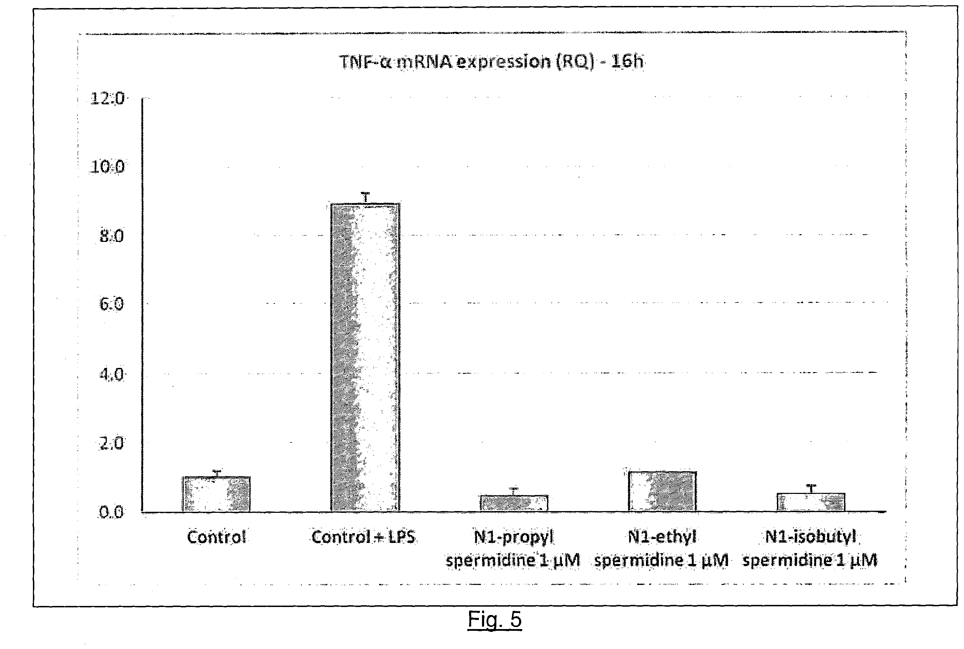

With reference to the graphs in Figures 4 and 5, samples of further compounds of the invention as specified below were subjected to anti-inflammatory test on an immortalized line of human keratinocytes NCTC2544 substantially as described above, to the general part whereof reference shall be made.

### Tested compounds

N¹-ethylspermidine, of formula

   (IV) H₂N-(CH₂)₃-N¹(C₂H₅)-(CH₂)₄-NH₂
N¹-propylspermidine, of formula

   (V) H₂N-(CH₂)₃-N¹(C₃H₇)-(CH₂)₄-NH₂
N¹-isobutylspermidine, of formula

   (VI) H₂N-(CH₂)₃-N¹(C₄H₉)-(CH₂)₄-NH₂

### Controls

The controls containing only culture medium (negative control) and culture medium plus LPS (5 µg/ml) (positive control), were included in each plate.

### Tests and results

The cells were exposed to the action of samples of the compounds N¹-ethylspermidine, N¹-propylspermidine, N¹-isobutylspermidine at the concentrations of 1 nM and 1 µM.

The results are shown in the graphs in Figures 4 and 5 of the accompanying drawings.

Fig. 4 shows the gene expression of TNF-α (mRNA expression RQ) on NCTC2544 cells treated with the samples in question at a concentration of 1 nM and the two controls as defined above, after 16 hours of treatment.

Fig. 5 shows the gene expression of TNF-α (mRNA expression RQ) on NCTC2544 cells treated with the samples in question at a concentration of 1 µM and the two controls as defined above, after 16 hours of treatment.

The gene expression data were obtained by RT-PCR. The data are interpreted as relative decrease compared to the treatment with LPS alone and the data expressed as RQ (relative quantification) or as a percentage are directly comparable.

In general, the results obtained show a remarkable anti-inflammatory effect of the compounds of the invention at all the examined concentrations.

The results are shown in the graphs in Figures 4 and 5 of the accompanying drawings.

Fig. 4 shows the gene expression of TNF-α (mRNA expression RQ) on NCTC2544 cells treated with the samples in question at a concentration of 1 nM and the two controls as defined above, after 16 hours of treatment.

Fig. 5 shows the gene expression of TNF-α (mRNA expression RQ) on NCTC2544 cells treated with the samples in question at a concentration of 1 µM and the two controls as defined above, after 16 hours of treatment.

The gene expression data were obtained by RT-PCR. The data are interpreted as relative decrease compared to the treatment with LPS alone and the data expressed as RQ (relative quantification) or as a percentage are directly comparable.

In general, the results obtained show a remarkable anti-inflammatory effect of the compounds of the invention at all the examined concentrations.

## Claims

1. Compounds of formula (I) R-N¹-spermidine, i.e. 1.4-butanediamine,N-(3-aminopropyl)-N¹-R,
(I) H₂N-(CH₂)₃-N¹(R)-(CH₂)₄-NH₂
wherein R is a substituent linked to the secondary amine function of spermidine, selected from:
methyl, ethyl, propyl, isobutyl,
or a corresponding pharmaceutically acceptable salt, for use in opposing the effects of skin aging through an anti-inflammatory action.

2. Compound for use according to claim 1 for opposing the effects of skin aging through an antioxidant and anti-inflammatory action.

3. Compound for use according to claims 1 and 2, wherein said compound of formula (I) is applied to the skin topically.

4. Compound for use according to claims 1 and 2, wherein said compound of formula (I) is N¹-methylspermidine, i.e. N-(3-aminopropyl)-N¹-methyl-1,4 butanediamine of formula:
(II) H₂N-(CH₂)₃-N¹(CH₃)-(CH₂)₄-NH₂

5. Compound for use according to claims 1 and 2, wherein said compound of formula (I) is N¹-ethylspermidine of formula
(IV) H₂N-(CH₂)₃-N¹(C₂H₅)-(CH₂)₄-NH₂

6. Compound for use according to claims 1 and 2, wherein said compound of formula (I) is N¹-propylspermidine of formula
(V) H₂N-(CH₂)₃-N¹(C₃H₇)-(CH₂)₄-NH₂

7. Compound for use according to claims 1 and 2, wherein said compound of formula (I) is N¹-isobutylspermidine of formula
(VI) H₂N-(CH₂)₃-N¹(C₄H₉)-(CH₂)₄-NH₂

8. Compound for use according to claims 1 and 2, wherein said compound of formula (I) is in the form of trichydrochloride salt or salt with maleic acid.

9. A composition for use in opposing the effects of skin aging through an anti-inflammatory action, wherein a compound of formula (I) R-N¹-spermidine, i.e. 1,4-butanediamine,N-(3-aminopropyl)-N¹-R,
(I) H₂N-(CH₂)₃-N¹(R)-(CH₂)₄-NH₂
wherein R is a substituent linked to the secondary amine function of spermidine, selected from:
methyl, ethyl, propyl, isobutyl or a corresponding pharmaceutically acceptable salt, is formulated with excipients suitable for topical administration on the skin.

10. Composition for use according to claim 9, wherein said compound of formula (I) is N¹-methylspermidine, i.e. N-(3-aminopropyl)-N¹-methyl-1,4 butanediamine of formula:
(II) H₂N-(CH₂)₃-N¹(CH₃)-(CH₂)₄-NH₂

11. Composition for use according to claim 9, wherein said compound of formula (I) is N¹-ethylspermidine of formula
(IV) H₂N-(CH₂)₃-N¹(C₂H₅)-(CH₂)₄-NH₂

12. Composition for use according to claim 9, wherein said compound of formula (I) is N¹-propylspermidine of formula
(V) H₂N-(CH₂)₃-N¹(C₃H₇)-(CH₂)₄-NH₂

13. Composition for use according to claim 9, wherein said compound of formula (I) is
N¹-isobutylspermidine of formula
(VI) H₂N-(CH₂)₃-N¹(C₄H₉)-(CH₂)₄-NH₂

14. Composition for use according to claim 9, wherein said compound of formula (I) is present in an amount, in percentage by weight (w/w%), from 0.0001 to 0.30.

15. Composition for use according to claim 14, wherein said compound of formula (I) is present in an amount, in percentage by weight (w/w%), from 0.010 to 0.30.

16. Composition for use according to claim 14, wherein said compound of formula (I) is present in an amount, in percentage by weight (w/w%), from 0.0001 to 0.15.

17. Compounds of formula (I) R-N¹-spermidine, i.e. 1.4-butanediamine,N-(3-aminopropyl)-N¹-R,
(I) H₂N-(CH₂)₃-N¹(R)-(CH₂)₄-NH₂
wherein R is a substituent linked to the secondary amine function of spermidine, selected from:
methyl, ethyl, propyl, isobutyl or a corresponding pharmaceutically acceptable salt, for use in the treatment of the effects of skin aging through an anti-inflammatory action.

18. Composition comprising a compound according to claim 17 for use in the treatment of the effects of skin aging through an anti-inflammatory action.

## Patentansprüche

1. Zusammensetzungen der Formel (I) R-N¹- Spermidin, d.h. 1,4-Butandiamin,N-(3-Aminopropyl)-N¹-R,
(I) H₂N-(CH₂)₃-N¹(R)-(CH₂)₄-NH₂
wobei R ein Substituent ist, der an die sekundäre Aminfunktion von Spermidin gebunden ist und der ausgewählt ist aus:
Methyl, Ethyl, Propyl, Isobutyl,
oder einem korrespondierenden pharmazeutisch akzeptablen Salz, zur Verwendung bei der Bekämpfung der Auswirkungen von Hautalterung mittels einer entzündungshemmenden Wirkung.

2. Zusammensetzung zur Verwendung gemäß Anspruch 1 zur Bekämpfung der Auswirkungen von Hautalterung mittels einer antioxidativen und entzündungshemmenden Wirkung.

3. Zusammensetzung zur Verwendung gemäß den Ansprüchen 1 und 2, wobei die Zusammensetzung der Formel (I) topisch auf die Haut aufgetragen wird.

4. Zusammensetzung zur Verwendung gemäß den Ansprüchen 1 und 2, wobei die Zusammensetzung der Formel (I) N¹-Methylspermidin ist, d. h.
N-(3-Aminopropyl)-N¹-Methyl-1,4-Butandiamin der Formel
(II) H₂N-(CH₂)₃-N¹(CH₃)-(CH₂)₄-NH₂

5. Zusammensetzung zur Verwendung gemäß den Ansprüchen 1 und 2, wobei die Zusammensetzung der Formel (I) N¹-Ethylspermidin der Formel
(IV) H₂N-(CH₂)₃-N¹(C₂H₅)-(CH₂)₄-NH₂ ist.

6. Zusammensetzung zur Verwendung gemäß den Ansprüchen 1 und 2, wobei die Zusammensetzung der Formel (I) N¹-Propylspermidin der Formel
(V) H₂N-(CH₂)₃-N¹(C₃H₇)-(CH₂)₄-NH₂ ist.

7. Zusammensetzung zur Verwendung gemäß den Ansprüchen 1 und 2, wobei die Zusammensetzung der Formel (I) N¹ - Isobutylspermidin der Formel
(VI) H₂N-(CH₂)₃-N¹(C₄H₉)-(CH₂)₄-NH₂ ist.

8. Zusammensetzung zur Verwendung gemäß den Ansprüchen 1 und 2, wobei die Zusammensetzung der Formel (I) in Form von Trihydrochlorid-Salz oder Salz mit Maleinsäure vorliegt.

9. Zusammensetzung zur Verwendung bei der Bekämpfung der Auswirkungen von Hautalterung mittels einer entzündungshemmenden Wirkung, mit einer Zusammensetzung der Formel (I) R-N¹-Spermidin, d. h. 1,4-Butandiamin,N-(3-Aminopropyl)-N¹-R,
(I) H₂N-(CH₂)₃-N¹(R)-(CH₂)₄-NH₂
wobei R ein Substituent ist, der an die sekundäre Aminfunktion von Spermidin gebunden ist und der ausgewählt ist aus:
Methyl, Ethyl, Propyl, Isobutyl oder einem korrespondierenden pharmazeutisch akzeptablen Salz, und mit Hilfsstoffen formuliert wird, die für die topische Verabreichung geeignet sind.

10. Zusammensetzung zur Verwendung gemäß Anspruch 9, wobei die Zusammensetzung der Formel (I) N¹-Methylspermidin ist, d.h. N-(3-Aminopropyl)-N¹-Methyl-1,4-Butandiamin der Formel
(II) H₂N-(CH₂)₃-N¹(CH₃)-(CH₂)₄-NH₂

11. Zusammensetzung zur Verwendung gemäß Anspruch 9, wobei die Zusammensetzung der Formel (I) N¹-Ethylspermidin der Formel
(IV) H₂N-(CH₂)₃-N¹(C₂H₅)-(CH₂)₄-NH₂ ist.

12. Zusammensetzung zur Verwendung gemäß Anspruch 9, wobei die Zusammensetzung der Formel (I) N¹-Propylspermidin der Formel
(V) H₂N-(CH₂)₃-N¹(C₃H₇)-(CH₂)₄-NH₂ ist.

13. Zusammensetzung zur Verwendung gemäß Anspruch 9, wobei die Zusammensetzung der Formel (I) N¹-Isobutylspermidin der Formel
(VI) H₂N-(CH₂)₃-N¹(C₄H₉)-(CH₂)₄-NH₂ ist.

14. Zusammensetzung zur Verwendung gemäß Anspruch 9, wobei die Zusammensetzung der Formel (I) in einer Menge zwischen 0,0001 und 0,30 Gew.-% vorliegt.

15. Zusammensetzung zur Verwendung gemäß Anspruch 14, wobei die Zusammensetzung der Formel (I) in einer Menge zwischen 0,010 und 0,30 Gew.-% vorliegt.

16. Zusammensetzung zur Verwendung gemäß Anspruch 14, wobei die Zusammensetzung der Formel (I) in einer Menge zwischen 0,0001 und 0,15 Gew.-% vorliegt.

17. Zusammensetzungen der Formel (I) R-N¹-Spermidin, d.h. 1,4-Butandiamin,N-(3-Aminopropyl)-N¹-R,
(I) H₂N-(CH₂)₃-N¹(R)-(CH₂)₄-NH₂
wobei R ein Substituent ist, der an die sekundäre Aminfunktion von Spermidin gebunden ist und der ausgewählt ist aus:
Methyl, Ethyl, Propyl, Isobutyl oder einem korrespondierenden pharmazeutisch akzeptablen Salz, zur Verwendung bei der Behandlung der Auswirkungen von Hautalterung mittels einer entzündungshemmenden Wirkung.

18. Zusammensetzung, aufweisend eine Zusammensetzung gemäß Anspruch 17 zur Verwendung bei der Behandlung der Auswirkungen von Hautalterung mittels einer entzündungshemmenden Wirkung.

## Revendications

1. Composés de formule (I) R-N¹-spermidine, c'est-à-dire un 1,4-butanediamine,N-(3-aminopropyl)-N¹-R,
(I) H₂N-(CH₂)₃-N¹(R)-(CH₂)₄-NH₂
dans laquelle R est un substituant lié à la fonction amine secondaire de la spermidine, sélectionné parmi :
un méthyle, un éthyle, un propyle, un isobutyle,
ou un sel pharmaceutiquement acceptable correspondant, pour une utilisation pour contrer les effets du vieillissement de la peau par une action anti-inflammatoire.

2. Composé pour une utilisation selon la revendication 1 pour contrer les effets du vieillissement de la peau par une action antioxydante et anti-inflammatoire.

3. Composé pour une utilisation selon les revendications 1 et 2, dans lequel ledit composé de formule (I) est appliqué sur la peau par voie topique.

4. Composé pour une utilisation selon les revendications 1 et 2, dans lequel ledit composé de formule (I) est la N¹-méthylspermidine, c'est-à-dire la N-(3-aminopropyl)-N¹-méthyl-1,4-butanediamine de formule
(II) H₂N-(CH₂)₃-N¹(CH₃)-(CH₂)₄-NH₂.

5. Composé pour une utilisation selon les revendications 1 et 2, dans lequel ledit composé de formule (I) est la N¹-éthylspermidine de formule
(IV) H₂N-(CH₂)₃-N¹(C₂H₅)-(CH₂)₄-NH₂.

6. Composé pour une utilisation selon les revendications 1 et 2, dans lequel ledit composé de formule (I) est la N¹-propylspermidine de formule
(V) H₂N-(CH₂)₃-N¹(C₃H₇)-(CH₂)₄-NH₂.

7. Composé pour une utilisation selon les revendications 1 et 2, dans lequel ledit composé de formule (I) est la N¹-isobutylspermidine de formule
(VI) H₂N-(CH₂)₃-N¹(C₄H₉)-(CH₂)₄-NH₂.

8. Composé pour une utilisation selon les revendications 1 et 2, dans lequel ledit composé de formule (I) est sous la forme d'un sel de trichlorhydrate ou d'un sel avec l'acide maléique.

9. Composition pour une utilisation pour contrer les effets du vieillissement de la peau par une action anti-inflammatoire, dans laquelle un composé de formule (I) R-N¹-spermidine, c'est-à-dire un 1,4-butanediamine,N-(3-aminopropyl)-N¹-R,
(I) H₂N-(CH₂)₃-N¹(R)-(CH₂)₄-NH₂
dans laquelle R est un substituant lié à la fonction amine secondaire de la spermidine, sélectionné parmi :
un méthyle, un éthyle, un propyle, un isobutyle ou un sel pharmaceutiquement acceptable correspondant, est formulé avec des excipients appropriés pour une administration topique sur la peau.

10. Composition pour une utilisation selon la revendication 9, dans laquelle ledit composé de formule (I) est la N¹-méthylspermidine, c'est-à-dire la N-(3-aminopropyl)-N¹-méthyl-1,4-butanediamine de formule :
(II) H₂N-(CH₂)₃-N¹(CH₃)-(CH₂)₄-NH₂.

11. Composition pour une utilisation selon la revendication 9, dans laquelle ledit composé de formule (I) est la N¹-éthylspermidine de formule
(IV) H₂N-(CH₂)₃-N¹(C₂H₅)-(CH₂)₄-NH₂.

12. Composition pour une utilisation selon la revendication 9, dans laquelle ledit composé de formule (I) est la N¹-propylspermidine de formule
(V) H₂N-(CH₂)₃-N¹(C₃H₇)-(CH₂)₄-NH₂.

13. Composition pour une utilisation selon la revendication 9, dans laquelle ledit composé de formule (I) est la N¹-isobutylspermidine de formule
(VI) H₂N-(CH₂)₃-N¹(C₄H₉)-(CH₂)₄-NH₂.

14. Composition pour une utilisation selon la revendication 9, dans laquelle ledit composé de formule (I) est présent en une quantité, en pourcentage en poids (% en poids/poids), de 0,0001 à 0,30.

15. Composition pour une utilisation selon la revendication 14, dans laquelle ledit composé de formule (I) est présent en une quantité, en pourcentage en poids (% en poids/poids), de 0,010 à 0,30.

16. Composition pour une utilisation selon la revendication 14, dans laquelle ledit composé de formule (I) est présent en une quantité, en pourcentage en poids (% en poids/poids), de 0,0001 à 0,15.

17. Composés de formule (I) R-N¹-spermidine, c'est-à-dire un 1,4-butanediamine,N-(3-aminopropyl)-N¹-R,
(I) H₂N-(CH₂)₃-N¹(R)-(CH₂)₄-NH₂
dans laquelle R est un substituant lié à la fonction amine secondaire de la spermidine, sélectionné parmi :
un méthyle, un éthyle, un propyle, un isobutyle ou un sel pharmaceutiquement acceptable correspondant, pour une utilisation dans le traitement des effets du vieillissement de la peau par une action anti-inflammatoire.

18. Composition comprenant un composé selon la revendication 17 pour une utilisation dans le traitement des effets du vieillissement de la peau par une action anti-inflammatoire.
